# EUROPEAN PATENT SPECIFICATION

(11) **EP 3 725 823 B1**
(45) Date of publication and mention of the grant of the patent: **10.09.2025**
(21) Application number: 18887770.8
(22) Date of filing: 23.11.2018
(51) Int. Cl.: C08H 7/00, C07D 307/50, C07D 307/46, C08H 8/00, C08L 97/00

(54) **METHOD FOR PRODUCING HIGH-ACTIVITY LIGNIN AND BY-PRODUCT FURFURAL AND APPLICATION**
VERFAHREN ZUR HERSTELLUNG VON HOCHAKTIVEM LIGNIN UND NEBENPRODUKT FURFURAL UND ANWENDUNG
PROCÉDÉ DE PRODUCTION DE LIGNINE À HAUTE ACTIVITÉ ET DE FURFURAL EN TANT QUE SOUS-PRODUIT ET APPLICATION

(30) Priority: 13.12.2017 CN 201711328316
(43) Date of publication of application: 21.10.2020
(73) Proprietor: JINAN SHENGQUAN GROUP SHARE-HOLDING CO., LTD., Jinan, Shandong 250204 (CN)
(72) Inventor: JIANG, Chengzhen, Jinan, Shandong 250204 (CN); GAO, Zhaofeng, Jinan, Shandong 250204 (CN); SHI, Feng, Jinan, Shandong 250204 (CN)
(74) Representative: Ezcurra Zufia, Maria Antonia
(86) International application number: PCT/CN2018/117273
(87) International publication number: WO 2019/114527

(56) References cited:
- WO-A1-2017/181280
- WO-A1-2017/181280
- CN-A- 103 193 737
- CN-A- 104 522 875
- CN-A- 105 264 080
- CN-A- 105 274 894
- CN-A- 108 017 790

## Description

### Technical Field

This invention belongs to the technical field of biomass, and relates to a method for producing high-activity lignin and by-product furfural and application thereof.

### Background Art

As an important organic chemical raw material and chemical solvent, furfural is widely used in the industries such as petroleum, chemicals, medicine, food, synthetic rubber and synthetic resin, and the global demand for furfural is huge every year. It can selectively extract unsaturated components from petroleum and vegetable oils, and can also extract aromatic components from lubricants and diesel oil. With the intensification of the energy crisis, the use of renewable agricultural and forestry wastes to produce high value-added furfural, and the development and utilization of its downstream chemical products have received increasing attention. China's furfural takes up a significant position in the world furfural trade, and has made great progress in recent years.

The production of furfural is based on the chemical conversion of pentose. At present, on the industrial scale, the only way to obtain furfural is still hydrolysis with corn cobs as the raw material. According to the principle of hydrolysis and the process of generating furfural, the methods for preparing furfural can be divided into direct method and indirect method. The direct method (one-step method) is to put the hemicellulose-containing raw materials into the hydrolysis pot, and under the action of catalyst and heat, the hemicellulose is hydrolyzed to pentose, and the pentose is dehydrated to generate furfural. The indirect method (two-step method) includes two steps, namely, hydrolysis reaction of hemicellulose and dehydration reaction of pentose, which are completed in different kinds of equipment.

CN 103261184 A discloses a method for preparing furfural from lignocellulose feedstock containing dextran and xylan, which makes the feedstock contact water in the presence of an acid catalyst, and then makes the obtained mixture contact at least one water-immiscible organic solvent to form a mixture containing an aqueous phase and an organic phase, and furfural is prepared under suitable reaction conditions, and preferably distributed into the organic phase from which furfural can be recovered.

CN 104072450 A discloses a method for preparing 5-hydroxymethyl furfural and furfural from biomass raw materials, comprising: adding biomass raw materials, solvent, solid phosphate catalyst and soluble inorganic salt to a reactor to form a reaction system. Under the protection of inert gas within the temperature range of 20-400°C, carry out dehydration reaction for 0-100h to obtain 5-hydroxymethyl furfural and furfural products with a higher yield.

None of the above patents can achieve the joint production of lignin and furfural, and the furfural extraction by two-phase extraction has a low yield and a complicated process. It is of important research significance to further improve the yield of furfural and achieve the joint production of lignin at the same time.

In the production process of pre-hydrolyzed sulfate dissolving pulp, the first step is pre-hydrolysis, which is carried out under acidic conditions. Generally, hemicellulose undergoes acidic hydrolysis and dissolves under the condition of acetic acid produced by steam high temperature self-hydrolysis. In this way, the content of hemicellulose (especially pentosan) in the raw material is greatly reduced. While hemicellulose undergoes acid hydrolysis, part of the lignin also dissolves under acidic conditions.

At present, after most of the pre-hydrolysate is concentrated by dissolving pulp production enterprises, it is added to an alkali recovery boiler for incineration, which not only wastes steam energy, but also increases the load of the alkali recovery boiler, thereby increasing the production cost of the dissolving pulp. The main components in the pre-hydrolysate are pentosan and lignin. It is of great significance to further use it comprehensively, turn waste into treasure, increase the capacity of the existing equipment of the dissolving pulp production enterprises, and reduce the production cost of dissolving pulp.

### Summary of the Invention

In view of the said shortcomings in the prior art, the object of this invention is to provide a method for producing high-activity lignin and by-product furfural and an application. The said method further increases the yield of the obtained lignin and furfural by catalyzing the dissolving pulp pre-hydrolysate and/or the sulfite cooking liquor with acid uses acid to catalyze dissolving pulp pre-hydrolysate and/or sulfite cooking liquor to obtain the lignin and furfural, in particular, the ratio of 5-methyl furfural in furfural to furfural is in a proper range, so that the strength of furan resin prepared from furfural can be further improved.

To achieve the above object, this invention provides the method of claim 1.

In this invention, dissolving pulp pre-hydrolysate and/or sulfite cooking liquid are used as specific raw materials, and acid is used for catalysis for joint production of lignin and furfural, which improves the yield of lignin and furfural.

The following are preferred technical solutions of this invention, but not as limitations to the technical solutions provided by this invention. Through the following preferred technical solutions, the technical objects and beneficial effects of this invention can be better achieved.

According to this invention, the said dissolving pulp pre-hydrolysate and/or sulfite cooking liquor contains lignin and pentosan.

According to this invention, the content of the said lignin is

According to this invention, the content of the said pentosan is 1-60%, for example, it can be 1%, 2%, 3%, 5%, 6%, 8%, 10%, 12%, 15%, 16%, 18%, 20%, 22%, 23%, 25%, 26%, 28%, 30%, 32%, 35%, 36%, 38%, 40%, 42%, 45%, 48%, 50%, 52%, 55%, 56%, 58% or 60%, preferably 1-30%, further preferably 1-10%.

According to this invention, the said acid is a mixture of sulfuric acid and phosphoric acid.

In this invention, the inventors unexpectedly discovered that compared to the use of one single acid for catalysis, the content of 5-methyl furfural in the produced furfural accounts for about 5% of the said furfural by using mixed acid for catalysis, so that the strength of furan resin prepared from such furfural is further improved, which is conducive to the subsequent comprehensive utilization of furfural.

According to this invention, the said mixed acid is a mixture of sulfuric acid and phosphoric acid, and the volume ratio of sulfuric acid to phosphoric acid is 1: (0.5-3), for example, it can be 1:0.5, 1:0.8, 1:1, 1:1.2, 1:1.5, 1:1.8, 1:2, 1:2.2, 1:2.5, 1:2.8 or 1:3, preferably 1: (0.8-1.5).

In this invention, the inventors found that the mixture of sulfuric acid and phosphoric acid has the best catalytic effect, especially when the volume ratio of sulfuric acid to phosphoric acid is in the range of 1:(0.5-3), and the furan resin has the highest strength when it is prepared from furfural obtained with such volume ratio within the range of 1:(0.8-1.5).

Compared with the prior art, this invention has the following beneficial effects.
(1) According to the method of this invention, dissolving pulp pre-hydrolysate and/or sulfite cooking liquid is used as the raw material, and mixed acid is used as a catalyst for catalysis and joint production of lignin and furfural.
(2) The furfural prepared with this invention can be used to prepare furan resin. Compared with furan resin prepared directly from furfural or a mixture of furfural and 5-methyl furfural, such furan resin has increased bonding strength, high strength, good stability at high temperatures, and very broad application prospects;
(3) The lignin prepared with the invention has good activity, and can be further used for preparing furan resin or phenol resin, etc., which is conducive to the subsequent comprehensive utilization of lignin.

### Detailed Description of the Preferred Embodiments

The technical terms mentioned in this specification have the same meanings as those generally understood by those skilled in the art. In case of inconsistency, the definitions in this specification shall prevail.

In this specification, the dissolving pulp pre-hydrolysate refers to the liquid containing pentosan, lignin, inorganic substances and other substances obtained through pre-hydrolyzing plant raw materials in the first step in the production process of dissolving pulp with the "pre-hydrolytic sulfate process".

In this specification, sulfite cooking liquid refers to the liquid containing lignosulfonates, carbohydrates and their degradation products, inorganic substances and other substances (commonly known as "red liquid") obtained through cooking the plant raw materials with sulfite in the production process of papermaking or dissolving pulp with the "sulfite process".

In this specification, pentosan, also known as pentose, refers to the general term of polymers composed of five-carbon sugars in hemicellulose, and various plant raw materials contain varying amounts of pentosan.

In this specification, heating medium refers to a carrier of heat. In industry, a heat transfer medium that transfers heat energy from a heating device to a heat-using unit in an indirect manner is called heating medium.

The technical solutions of this invention are further described below through specific embodiments.

This invention will be further described in detail in combination with the following embodiments. The embodiments described below are only preferred ones of this invention, and are not intended to limit this invention in other forms.

In the embodiments, the dissolving pulp pre-hydrolysate comes from Sappi, South Africa.

### Embodiment 1 - Preparation of lignin and furfural (I)

This embodiment is shown as a comparative or as a reference embodiment. The method for joint production of lignin and furfural specifically includes the following steps:
(1) Concentrate the dissolving pulp pre-hydrolysate to the reaction liquid with a solid content of 15%, wherein the pentosan content is 9.39%, the lignin content is 5.42%, and the ash content is 0.19%;
(2) Add 100g isophorone to the 250ml high-pressure reactor, stir at a rate of 100rppm, heat to 180°C, then add sulfuric acid catalyst that accounts for 0.8% of the mass of the reaction liquid to the reaction liquid, keep the temperature unchanged at 180°C, add the reaction liquid to the reactor at a rate of 10g/min, and distill out the distillate at the same time, control the distillation valve to make the reactor pressure at 0.2Mpa, maintain the moisture content in the reaction liquid within 5%, distill and react for 40min, stop feeding, collect distillate and weigh a total of 140g, which is the said furfural aqueous solution;
(3) Make the reaction mixture further react at a pressure of 0.2 Mpa and a temperature of 180°C for 20 min, cool to room temperature, add water to precipitate and filter, wash multiple times, and the brownish yellow solid obtained after recovering isophorone is lignin.

### Embodiment 2 - Preparation of lignin and furfural (II)

This embodiment is shown as a comparative or as a reference embodiment

The method for joint production of lignin and furfural specifically includes the following steps:
(1) Concentrate the dissolving pulp pre-hydrolysate to the reaction liquid with a solid content of 20%, wherein the pentosan content is 12.48%, the lignin content is 7.21%, and the ash content is 0.31 %;
(2) Add 100g sulfolane to the 250ml high-pressure reactor, stir at a rate of 150rppm, heat to 170°C, then add phosphoric acid catalyst that accounts for 1.0% of the mass of the reaction liquid to the reaction liquid, keep the temperature unchanged at 170°C, add the reaction liquid to the reactor at a rate of 10g/min, and distill out the distillate at the same time, control the distillation valve to make the reactor pressure at 0.3Mpa, maintain the moisture content in the reaction liquid within 3%, distill and react for 40min, stop feeding, collect distillate and weigh a total of 132g, which is the said furfural aqueous solution;
(3)Make the reaction mixture further react at a pressure of 0.3 Mpa and a temperature of 170°C for 20 min, cool to room temperature, add water to precipitate and filter, wash multiple times, and the brownish yellow solid obtained after recovering sulfolane is lignin.

### Embodiment 3 - Preparation of lignin and furfural (III)

This embodiment is shown as a comparative or as a reference embodiment.

The method for joint production of lignin and furfural specifically includes the following steps:
(1) Concentrate the dissolving pulp pre-hydrolysate to the reaction liquid with a solid content of 8%, wherein the pentosan content is 9.39%, the lignin content is 5.42%, and the ash content is 0.19%;
(2) Add 100g gamma-valerolactone to the 250ml four-necked bottle, stir at a rate of 200rppm, vacuum the four-necked bottle to -0.1Mpa, heat to 200°C, then add hydrochloric acid catalyst that accounts for 0.1% of the mass of the reaction liquid to the reaction liquid, keep the temperature unchanged at 200°C, add the reaction liquid to the four-necked bottle at a rate of 10g/min, and obtain the distillate through vacuum distillation at the same time, make the pressure of the four-necked bottle at -0.1 Mpa, maintain the moisture content in the reaction liquid within 5%, distill and react for 90min, stop feeding, collect distillate and weigh a total of 126g, which is the said furfural aqueous solution;
(3) Make the reaction mixture further react at a pressure of -0.1 Mpa and a temperature of 200°C for 30 min, cool to room temperature, add water to precipitate and filter, wash multiple times, and the brownish yellow solid obtained after recovering gamma-valerolactone is lignin.

### Embodiment 4 - Preparation of lignin and furfural (IV)

This embodiment is shown as a comparative or as a reference embodiment.

The method for joint production of lignin and furfural specifically includes the following steps:
(1) Concentrate the sulfite cooking liquid to the reaction liquid with a solid content of 40%, wherein the pentosan content is 12.48%, the lignin content is 7.21%, and the ash content is 0.31%;
(2) Add 100g propylene carbonate to the 250ml high-pressure reactor, stir at a rate of 50rppm, heat to 160°C, then add acetic acid catalyst that accounts for 9.6% of the mass of the reaction liquid to the reaction liquid, keep the temperature unchanged at 160°C, add the reaction liquid to the reactor at a rate of 10g/min, and distill out the distillate at the same time, control the distillation valve to make the reactor pressure at 0.5Mpa, maintain the moisture content in the reaction liquid within 5%, distill and react for 40min, stop feeding, collect distillate and weigh a total of 162g, which is the said furfural aqueous solution;
(3) Make the reaction mixture further react at a pressure of 0.5 Mpa and a temperature of 160°C for 30 min, cool to room temperature, add water to precipitate and filter, wash multiple times, and the brownish yellow solid obtained after recovering propylene carbonate is lignin.

### Embodiment 5 - Preparation of lignin and furfural (V)

This embodiment is shown as a comparative or as a reference embodiment.

Compared with Embodiment 1, the acid in step (2) is a combination of sulfuric acid and hydrochloric acid, wherein the volume ratio of sulfuric acid to hydrochloric acid is 1:0.5, the added mixed acid accounts for 0.15% of the mass of the said reaction liquid, and the other components and steps are the same as those in Embodiment 1.

### Embodiment 6 - Preparation of lignin and furfural (VI)

This embodiment is shown as a comparative or as a reference embodiment.

Compared with Embodiment 1, the acid in step (2) is a combination of phosphoric acid and nitric acid, wherein the volume ratio of phosphoric acid to nitric acid is 1:3, the added mixed acid accounts for 0.8% of the mass of the said reaction liquid, and the other components and steps are the same as those in Embodiment 1.

### Embodiment 7 - Preparation of lignin and furfural (VII)

This embodiment is shown as a comparative or as a reference embodiment.

Compared with Embodiment 1, the acid in step (2) is a combination of sulfuric acid and acetic acid, wherein the volume ratio of sulfuric acid to acetic acid is 1:1, the added mixed acid accounts for 0.8% of the mass of the said reaction liquid, and the other components and steps are the same as those in Embodiment 1.

### Embodiment 8 - Preparation of lignin and furfural (VII)

Compared with Embodiment 1, the acid in step (2) is a combination of sulfuric acid and phosphoric acid, wherein the volume ratio of sulfuric acid to acetic acid is 1:1.2, the added mixed acid accounts for 0.8% of the mass of the said reaction liquid, and the other components and steps are the same as those in Embodiment 1.

### Comparative example 1

Compared with Embodiment 1, the raw material in step (1) is corn cobs, and the other components and steps are the same as those in Embodiment 1.

### Comparative example 2

Compared with Embodiment 1, the dissolving slurry pre-hydrolysate in step (1) is concentrated to the reaction liquid with a solid content of 5%, and the other components and steps are the same as those in Embodiment 1.

### Comparative example 3

Compared with Embodiment 1, the dissolving slurry pre-hydrolysate in step (1) is concentrated to the reaction liquid with a solid content of 43%, and the other components and steps are the same as those in Embodiment 1.

### Comparative example 4

Compared with Embodiment 1, the amount of acid added in step (2) accounts for 12% of the mass of the reaction liquid, and the other components and steps are the same as those in Embodiment 1.

### Test and analysis

The components of the lignin and furfural prepared in Embodiments 1-8 and Comparative Examples 1-4 were analyzed with liquid chromatography, and their physical and chemical properties were also analyzed. The results are shown in Table 1:

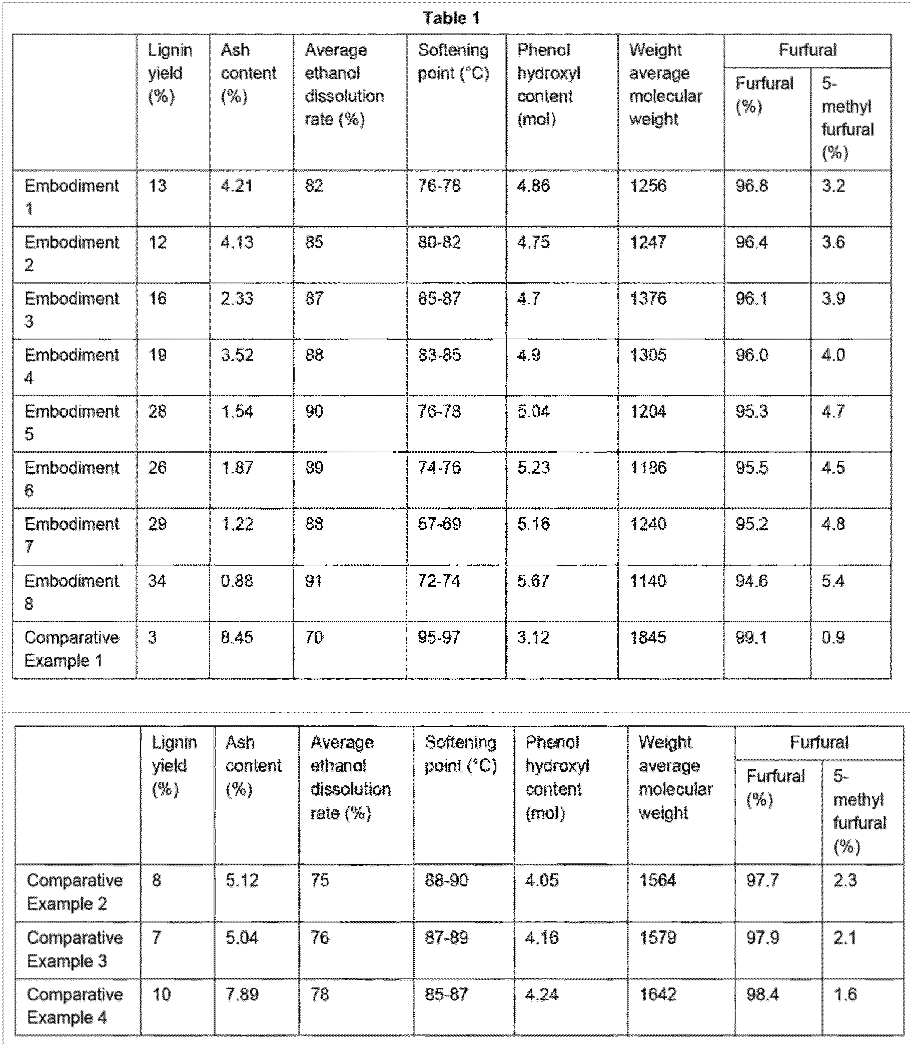

As can be seen from Table 1, in Embodiments 1-8, the yield of lignin from the furfural obtained through catalysis with the mixed acid can reach 34%, the ash content is low, the content of impurities is low, the average ethanol dissolution rate is high, the softening point is low, the content of phenolic hydroxyl group is large, and the weight average molecular weight is low. According to the comparison between Comparative Examples 1-4 and Embodiment 1, the lignin from the furfural prepared from other raw materials (such as corn cobs) is reduced, the ash content is high, the content of impurities is high, and the weight average molecular weight is high; moreover, the proportion of 5-methyl furfural in furfural is low. The concentration of solid content is not within the scope of this application or if the acid catalyst added is excessive, the yield of lignin from the prepared furfural will be reduced, the ash content will be high, the content of impurities will be high, the weight average molecular weight will be high, and the proportion of 5-methyl furfural in furfural will be low.

### Embodiment 9 - Synthesis of furan resin (I)

This embodiment is shown as a comparative or as a reference embodiment.

The synthesis method of the said furan resin includes the following steps:
(1) Take 900g furfural in Embodiment 1 and add it to the reaction kettle, add copper catalyst to the said reaction kettle for hydrogenation reaction, filter the catalyst, and distill furfuryl alcohol, and 900g furfuryl alcohol is obtained;
(2) Add 176g formaldehyde (concentration: 37%) to the reaction kettle, start stirring, add 80kg urea, adjust the pH value with alkaline solution to 8.8-9.0 after the urea is dissolved, raise the temperature to 90±2°C and react for 1 hour; add the said furfuryl alcohol of 200g and further react for 1 hour; adjust the pH value of the material system to 4.5-4.8, and react at a reaction temperature of 95±2°C for 1.5 hours;
(3) Add 20g urea, adjust the pH value of the material system to 7.8-8.0, react for 1 hour at a temperature of 75±2°C, cool the material system and stop the reaction; dehydrate 110g under vacuum, then add the said furfuryl alcohol of about 623g, stir evenly, discharge the material, and the obtained product is about 989g.

### Embodiment 10 - Synthesis of furan resin (II)

This embodiment is shown as a comparative or as a reference embodiment.

Compared with Embodiment 9, except that furfural in Embodiment 2 is used to prepare the said furan resin, the other components and steps are the same as those in Embodiment 9.

### Embodiment 11 - Synthesis of furan resin (III)

This embodiment is shown as a comparative or as a reference embodiment.

Compared with Embodiment 9, except that furfural in Embodiment 3 is used to prepare the said furan resin, the other components and steps are the same as those in Embodiment 9.

### Embodiment 12 - Synthesis of furan resin (IV)

This embodiment is shown as a comparative or as a reference embodiment.

Compared with Embodiment 9, except that furfural in Embodiment 4 is used to prepare the said furan resin, the other components and steps are the same as those in Embodiment 9.

### Embodiment 13 - Synthesis of furan resin (V)

This embodiment is shown as a comparative or as a reference embodiment.

Compared with Embodiment 9, except that furfural in Embodiment 5 is used to prepare the said furan resin, the other components and steps are the same as those in Embodiment 9.

### Embodiment 14 - Synthesis of furan resin (VI)

This embodiment is shown as a comparative or as a reference embodiment.

Compared with Embodiment 9, except that furfural in Embodiment 6 is used to prepare the said furan resin, the other components and steps are the same as those in Embodiment 9.

### Embodiment 15 - Synthesis of furan resin (VII)

This embodiment is shown as a comparative or as a reference embodiment.

Compared with Embodiment 9, except that furfural in Embodiment 7 is used to prepare the said furan resin, the other components and steps are the same as those in Embodiment 9.

### Embodiment 16 - Synthesis of furan resin (VIII)

Compared with Embodiment 9, except that furfural in Embodiment 8 is used to prepare the said furan resin, the other components and steps are the same as those in Embodiment 9.

### Comparative Example 5

Compared with Embodiment 9, except that furfural in Comparative Example 1 is used to prepare the said furan resin, the other components and steps are the same as those in Embodiment 9.

### Comparative Example 6

Compared with Embodiment 9, except that furfural in Comparative Example 2 is used to prepare the said furan resin, the other components and steps are the same as those in Embodiment 9.

### Comparative Example 7

Compared with Embodiment 9, except that furfural in Comparative Example 3 is used to prepare the said furan resin, the other components and steps are the same as those in Embodiment 9.

### Comparative Example 8

Compared with Embodiment 9, except that furfural in Comparative Example 4 is used to prepare the said furan resin, the other components and steps are the same as those in Embodiment 9.

### Comparative Example 9

Compared with Embodiment 9, except that furfural available commercially is used to prepare the said furan resin, the other components and steps are the same as those in Embodiment 9.

### Test and Analysis

Physical and chemical test was carried out for the furan resins prepared in Embodiments 9-16 and Comparative Examples 5-9, and the analysis method of nitrogen content and free formaldehyde was carried out in accordance with JBT7526-2008 Self-set Furan Resin for Foundry.

The test results are shown in Table 2:

**Table 2**

| | pH | Density | Viscosity | Free aldehyde | Nitrogen content |
|---|---|---|---|---|---|
| Embodiment 9 | 8.24 | 1.18 | 22.01 | 0.78 | 4.80 |
| Embodiment 10 | 8.23 | 1.18 | 22.12 | 0.78 | 4.81 |

| | pH | Density | Viscosity | Free aldehyde | Nitrogen content |
|---|---|---|---|---|---|
| Embodiment 11 | 8.23 | 1.17 | 22.:4 | 0.78 | 4.80 |
| Embodiment 12 | 8.26 | 1.17 | 22.15 | 0.78 | 4.76 |
| Embodiment 13 | 8.25 | 1.18 | 21.81 | 0.78 | 4.75 |
| Embodiment 14 | 8.24 | 1.19 | 21.83 | 0.78 | 4.76 |
| Embodiment 15 | 8.23 | 1.19 | 21.72 | 0.78 | 4.75 |
| Embodiment 16 | 8.22 | 1.19 | 21.65 | 0.78 | 4.73 |
| Comparative example 5 | 8.21 | 1.17 | 22.33 | 0.78 | 4.82 |
| Comparative example 6 | 8.21 | 1.18 | 22.34 | 0.78 | 4.81 |
| Comparative example 7 | 8.23 | 1.17 | 22.37 | 0.78 | 7.80 |
| Comparative example 8 | 8.22 | 1.18 | 22.35 | 0.78 | 4.82 |
| Comparative example 9 | 8.21 | 1.16 | 22.45 | 0.78 | 4.83 |

As can be seen from Table 2, the viscosity of the furan resins prepared in Embodiments 9-16 is lower than that in Comparative Examples 5-9, and there are no obvious differences in other physical and chemical properties.

A sand mixing test was carried out for the furan resins prepared in Embodiments 9-16 and Comparative Examples 5-9 to test the tensile strength. The method for testing the tensile strength of resin bonded sand at room temperature was carried out according to JBT 7526-2008 Self-set Furan Resin for Foundry.

The specific conditions of the first sand mixing test are as follows: room temperature: 12.1°C, humidity: 50.1%, resin addition amount: 1.0%, curing agent GC09 addition amount: 50%, the specific conditions of the second sand mixing test are as follows: room temperature: 8.2°C, humidity: 22.4%, resin addition amount: 1.0%, curing agent GC09 addition amount: 50%; the test results are shown in Table 3:

**Table 3**

| | The first tensile strength (standard sand) | | | The second tensile strength (standard sand) | | |
|---|---|---|---|---|---|---|
| | 50min | 4h | 24h | 30min | 4h | 24h |
| Embodiment 9 | 0.28 | 2.25 | 2.54 | 0.26 | 1.12 | 1.62 |
| Embodiment 10 | 0.27 | 2.28 | 2.49 | 0.25 | 1.11 | 1.59 |
| Embodiment 11 | 0.27 | 2.19 | 2.41 | 0.22 | 1.07 | 1.51 |
| Embodiment 12 | 0.26 | 2.16 | 2.37 | 0.23 | 1.06 | 1.46 |
| Embodiment 13 | 0.30 | 2.54 | 2.54 | 0.28 | 1.18 | 1.68 |
| Embodiment 14 | 0.31 | 2.53 | 2.62 | 0.28 | 1.19 | 1.71 |
| Embodiment 15 | 0.32 | 2.67 | 2.79 | 0.27 | 1.17 | 1.69 |
| Embodiment 16 | 0.35 | 2.87 | 2.98 | 0.30 | 1.23 | 1.75 |
| Comparative example 5 | 0.21 | 1.35 | 1.78 | 0.20 | 0.89 | 1.23 |
| Comparative example 6 | 0.25 | 1.86 | 1.92 | 0.22 | 0.95 | 1.38 |
| Comparative example 7 | 0.24 | 1.81 | 1.91 | 0.27 | 0.96 | 1.34 |
| Comparative example 8 | 0.23 | 1.72 | 1.89 | 0.30 | 0.99 | 1.29 |
| Comparative example 9 | 0.22 | 1.3 | 1.75 | 0.20 | 0.90 | 1.22 |

As can be seen from Table 3, the furfural obtained in this invention contains 5-methyl furfural, and the furfuryl alcohol after hydrogenation contains 5-methyl furfuryl alcohol. From the comparison of Embodiments 9-16, it can be seen that the strength of the furan resin prepared from furfural catalyzed with a mixed acid is higher than that of the furan resin prepared from furfural catalyzed with a single acid; according to the comparison between Comparative Examples 5-9 and Embodiment 9, the strength of the furan resin prepared with other raw materials (such as corn cobs) or commercially available furfural is obviously reduced, and the concentration of solid content is not within the scope of this application. The strength of the prepared furan resin is also significantly reduced. Excessive acid catalyst added will also affect the strength of the prepared furan resin; it can be seen that with respect to the tensile strength of synthetic furan resin, the tensile strength of furan resin made from furfural prepared in this invention is 20% higher than that of furan resin made from commercially available furfural

In summary, the furfural prepared with this invention can be used to prepare furan resin. Compared with furan resin prepared directly from furfural or a mixture of furfural and 5-methyl furfural, such furan resin has increased bonding strength, high strength, good stability at high temperatures, and very broad application prospects.

The applicants declare that the said embodiments are the preferred ones of this invention, which are not intended to limit the scope of protection as defined in the appended claims.

## Claims

1. - A method for producing lignin and by-product furfural, **characterized in that** the said method comprises: using dissolving pulp pre-hydrolysate and/or sulfite cooking liquor as the raw material, and catalyzing by using acid as a catalyst to obtain the lignin and furfural;
Wherein,
the said dissolving pulp pre-hydrolysate and/or sulfite cooking liquor contains lignin and pentosan;
the content of the said lignin is 1-10%;
the content of the said pentosan is 1-60%, more preferably 1-30%, further preferably 1-10%;
the said acid is a mixture of sulfuric acid and phosphoric acid;
the volume ratio of sulfuric acid to phosphoric acid is 1: (0.5-3), more preferably 1: (0.8-1.5),
**characterized in that** it includes the following steps:
(1) Concentrate the dissolving pulp pre-hydrolysate and/or sulfite cooking solution, add it to the heating medium, add acid, then mix and stir, react under suitable conditions, and distill to obtain furfural aqueous solution;
(2) Make the remaining feed liquid in step (1) further react, and precipitate and filter to obtain lignin,
Wherein,
the solid content after concentration in step (1) is 10-30%;
the heating medium described in step (1) is a liquid and/or solid with a boiling point or melting point greater than 160°C, more preferably a liquid and/or solid with a boiling point or melting point greater than 180°C;
the said liquid heating medium in step (1) is any one or a mixture of at least two of dimethyl sulfoxide, dimethyl sulfone, diethyl sulfone, diphenyl sulfone, gamma-valerolactone, polyethylene glycol, glycerin, 1,3- propylene glycol, sulfolane, isophorone and propylene carbonate;
the said solid heating medium in step (1) is any one or a mixture of at least two of carbon powder, granular salt, silica or rock powder.

2. The method according to any of Claim 1, **characterized in that** the added amount of the said acid accounts for 0.1-10% of the mass of the said reaction liquid, preferably 0.15-5%;
Preferably, the said stirring rate is 50-300 rpm, more preferably 100-200 rpm;
Preferably, the pressure of the said reaction in step (1) is -0.1-0.5 Mpa, more preferably -0.1-0.3 Mpa;
Preferably, the temperature of the said reaction in step (1) is 160-200°C, more preferably 170-190°C.

3. The method according to any of Claims 1-2, **characterized in that** the time of the said reaction in step (2) is less than 120 min, preferably not more than 90 min, further preferably 40-90 min;
Preferably, the pressure of the said reaction in step (2) is -0.1-0.5 Mpa, preferably-0.1-0.3Mpa;
Preferably, the temperature of the said reaction in step (2) is 160-200°C, more preferably 170-180°C.

4. The method according to any of Claims 1-3, **characterized in that** it includes the following steps:
(1) Concentrate the dissolving pulp pre-hydrolysate and/or sulfite cooking solution to 10-30% solid content, add it to the heating medium, add acid that accounts for 0.1-2% of the mass of the reaction liquid, then mix and stir at a stirring rate of 50-300 rpm, and react at a pressure of -0.1-0.5 Mpa and a temperature of 160-200°C, and distill to obtain furfural aqueous solution;
(2) Make the remaining liquid in step (1) further react at a pressure of -0.1-0.5 Mpa and a temperature of 160-200°C for less than 120 minutes, and then precipitate and filter to obtain lignin.

## Patentansprüche

1. Verfahren zur Herstellung von Lignin und dem Nebenprodukt Furfural, **dadurch gekennzeichnet, dass** das Verfahren umfasst: Verwendung von Chemiezellstoff-Vorhydrolysat und/oder Sulfitkochsäure als Rohmaterial und Katalyse unter Verwendung von Säure als Katalysator, um das Lignin und Furfural zu erhalten;
**Dadurch gekennzeichnet, dass**
das genannte Chemiezellstoff-Vorhydrolysat und/oder die Sulfitkochsäure Lignin und Pentosan enthalten;
der Gehalt an diesem Lignin 1-10 % beträgt;
der Gehalt an diesem Pentosan 1-60 %, besser noch 1-30 %, noch bevorzugter 1-10 % beträgt;
die genannte Säure eine Mischung aus Schwefelsäure und Phosphorsäure ist;
das Volumenverhältnis von Schwefelsäure zu Phosphorsäure 1: (0,5-3), besser noch 1: (0,8-1,5) beträgt, **dadurch gekennzeichnet, dass** es die folgenden Schritte umfasst:
(1) Das Chemiezellstoff-Vorhydrolysat und/oder der Sulfitkochsäure konzentrieren, dies zum Heizmedium hinzufügen, Säure hinzufügen, dann mischen und umrühren, unter geeigneten Bedingungen reagieren lassen und destillieren, um eine wässrige Furfural-Lösung zu erhalten; (2) Die verbleibende Speiseflüssigkeit in Schritt (1) weiter reagieren lassen und ausfällen und filtern, um Lignin zu erhalten,
**Dadurch gekennzeichnet, dass**
der Feststoffgehalt nach der Konzentration in Schritt (1) 10-30 % beträgt;
das in Schritt (1) beschriebene Heizmedium eine Flüssigkeit und/oder ein Feststoff mit einem Siede- oder Schmelzpunkt von mehr als 160 °C, besser noch eine Flüssigkeit und/oder ein Feststoff mit einem Siede- oder Schmelzpunkt von mehr als 180 °C ist;
dieses flüssige Heizmedium in Schritt (1) ein beliebiger oder ein Gemisch aus mindestens zwei der folgenden Stoffe ist: Dimethylsulfoxid, Dimethylsulfon, Diethylsulfon, Diphenylsulfon, Gamma-Valerolacton, Polyethylenglykol, Glycerin, 1,3-Propylenglykol, Sulfolan, Isophoron und Propylencarbonat;
dieses Feststoff-Heizmedium in Schritt (1) ein beliebiger oder ein Gemisch aus mindestens zwei der folgenden Stoffe ist: Kohlenstoffpulver, Salzgranulat, Kieselsäure oder Gesteinspulver.

2. Verfahren nach einem der Ansprüche 1, **dadurch gekennzeichnet, dass** die zugesetzte Menge der genannten Säure 0,1-10 % der Masse der Reaktionsflüssigkeit ausmacht, vorzugsweise 0,15-5 %;
Vorzugsweise beträgt die Rührgeschwindigkeit 50-300 min⁻¹, besser noch 100-200 min⁻¹;
Vorzugsweise beträgt der Druck der genannten Reaktion in Schritt (1) -0,1-0,5 MPa, besser noch -0,1-0,3 MPa;
Vorzugsweise beträgt die Temperatur der genannten Reaktion in Schritt (1) 160-200 °C, besser noch 170-190 °C;

3. Verfahren nach einem der Ansprüche 1 bis 2, **dadurch gekennzeichnet, dass** die Zeit der genannten Reaktion in Schritt (2) weniger als 120 min, vorzugsweise nicht mehr als 90 min, noch bevorzugter 40 bis 90 min beträgt;
Vorzugsweise beträgt der Druck der genannten Reaktion in Schritt (2) -0,1-0,5 MPa, besser noch -0,1-0,3 MPa;
Vorzugsweise beträgt die Temperatur der genannten Reaktion in Schritt (2) 160-200 °C, besser noch 170-190 °C;

4. Verfahren nach einem der Ansprüche 1 bis 3, **dadurch gekennzeichnet, dass** es folgende Schritte umfasst:
(1) Das Chemiezellstoff-Vorhydrolysat und/oder der Sulfitkochsäure auf 10-30 % Feststoffgehalt konzentrieren, dies zum Heizmedium hinzufügen, Säure hinzufügen, die 0,1-2 % der Masse der Reaktionsflüssigkeit ausmacht, dann mischen und bei einer Rührgeschwindigkeit von 50-300 min⁻¹ rühren und bei einem Druck von -0,1-0,5 MPa und einer Temperatur von 160-200 °C reagieren lassen und destillieren, um eine wässrige Furfural-Lösung zu erhalten;
(2) Die in Schritt (1) verbleibende Flüssigkeit bei einem Druck von -0,1-0,5 MPa und einer Temperatur von 160-200 °C für weniger als 120 Minuten weiter reagieren lassen und dann ausfällen und filtern, um Lignin zu erhalten.

## Revendications

1. - Un procédé de production de lignine et de furfural comme sous-produit, **caractérisé par le fait que** ledit procédé comprend ; l'utilisation d'un pré-hydrolysat de pâte à dissoudre et/ou d'une liqueur de sulfite de cuisson comme matière première, et la catalyse par l'acide comme catalyseur pour obtenir la lignine et le furfural ;
où,
ledit pré-hydrolysat de pâte à dissoudre et/ou la liqueur de sulfite de cuissoncontient de la lignine et du pentosan ;la teneur en lignine est de 1 à 10% ;la teneur en pentosan est de 1 à 60%, de préférence de 1 à 30%, et encore plus préférablement de 1 à 10% ;
ledit acide est un mélange d'acide sulfurique et d'acide phosphorique ;le rapport volumique acide sulfurique - acide phosphorique est de 1 : (0,5-3), de préférence 1: (0,8-1,5),5),**caractérisé par le fait qu'**il comprend les étapes suivantes
(1) Concentrer le pré-hydrolysat de pâte à dissoudre et/ou la solution de sulfite de cuisson,l'ajouter au milieu chauffant, ajouter de l'acide, puis mélanger et agiter, laisser réagir dans des conditions adéquates et le distiller pour obtenir une solution aqueuse de furfural ;
(2) Laisser réagir le liquide résiduel issu de l'étape (1) et le précipiter et le filtrerpour obtenir la lignine,
où,
le contenu solide après concentration à l'étape (1) est 10-30% ;
le milieu de chauffage décrit à l'étape (1) est un liquide et/ou un solide avec un point d'ébullition ou un point de fusion supérieur à 160°C, de préférence un liquide et/ou un solide avec un point d'ébullition ou un point de fusion supérieur à 180°C ;
ledit milieu de chauffage liquide à l'étape (1) est un ou un composé d'au moins deux des substances suivantes : diméthylsulfoxyde, diméthylsulfone, diéthylsulfone, diphénylsulfone, gamma-valérolactone, polyéthylène glycol, glycérine, 1,3-propanediol, sulfolane, isophorone, carbonate de propylène ;
ledit milieu de chauffage solide à l'étape (1) est un composé unique ou un mélange d'au moins deux des matières suivantes : poudre de carbone, sel granulé, silice ou poudre de roche.

2. Le procédé conformément à l'une des revendications 1, **caractérisé par le fait que** la quantité ajoutée dudit acide représente 0,1 - 10% de la masse dudit liquide de réaction, de préférence 0,15 - 5% ;
De préférence, la vitesse d'agitation est de 50-300 t/min, préférablement 100-200 t/min ;
De préférence, la pression de ladite réaction à l'étape (1) est de -0,1-0,5 Mpa, préférablement -0,1-0,3 Mpa ;
De préférence, la température de ladite réaction à l'étape (1) est de 160-200°C, préférablement 170- 190°C.

3. Le procédé, conformément à l'une des revendications 1-2, **caractérisé par le fait que** la durée de ladite réaction à l'étape (2) est inférieure à 120 min, préférablement pas plus de 90 min, encore préférablement 40 à 90 min ;
De préférence, la pression de ladite réaction à l'étape (2) est de -0,1-0,5 Mpa, préférablement -0,1-0,3 Mpa ;
De préférence, la température de ladite réaction à l'étape (2) est de 160-200°C, préférablement 170- 180°C.

4. Le procédé, conformément à l'une des revendications 1-3, **caractérisé par le fait qu'**il comprend les étapes suivantes :
(1) Concentrer le pré-hydrolysat de pâte à dissoudre et/ou la solution de sulfite de cuisson à 10-30% de contenu solide, l'ajouter au milieu chauffant, ajouter de l'acide qui représente de 0,1 à 2% de la masse du liquide de réaction, puis mélanger et agiter à une vitesse de 50 à 300 t/min, et laisser réagir à une pression de -0,1-0,5 Mpa et une température de 160-200°C, et distiller pour obtenir la solution aqueuse de furfural ;
(2) Laisser le liquide résiduel de l'étape (1) réagir à une pression de -0,1-0,5 Mpa et à une température de 160-200°C pendant moins de 120 minutes, puisque le faire précipiter et le filtrer pour obtenir de la lignine.
